# FASCICULE DE BREVET EUROPEEN

(11) **EP 1 505 928 B1**
(45) Date de publication et mention de la délivrance du brevet: **05.04.2006**
(21) Numéro de dépôt: 03752850.2
(22) Date de dépôt: 22.05.2003
(51) Int. Cl.: A61F 2/00

(54) **Ensemble de soutènement sous-urétral dans le traitement de l'incontinence urinaire d'effort de la femme**
Suburethrale Stützanordnung zur Behandlung der weiblichen stressbedingten Harninkontinenz
Suburethral support assembly for the treatment of female stress urinary incontinence

(30) Priorité: 22.05.2002 US 151982
(43) Date de publication de la demande: 16.02.2005
(73) Titulaire: SOFRADIM PRODUCTION, 01600 Trévoux (FR)
(72) Inventeur: THERIN, Michel, F-69004 Lyon (FR)
(74) Mandataire: Brédeville, Odile Marie
(86) Numéro de dépôt international: PCT/FR2003/001554
(87) Numéro de publication internationale: WO 2003/096928

(56) Documents cités:
- WO-A-00/74633
- WO-A-02/098322
- DE-C- 219 999
- FR-A- 2 814 939
- US-B1- 6 491 703

## Description

La présente invention concerne le traitement chirurgical de l'incontinence urinaire d'effort de la femme.

Plus particulièrement, l'invention concerne un ensemble chirurgical permettant, au terme de l'intervention ou opération, de soutenir l'urètre au moyen d'une bandelette sous-urétrale, dont les deux extrémités sont chacune disposée dans un trajet approprié du corps de la patiente, par exemple dans l'espace rétropubien vers la paroi abdominale de celle-ci.

Il est connu de FR 2 814 939 un ensemble chirurgical comprenant une bande composite rassemblant une bandelette, une gaine et une aiguille, la bande composite comprenant une partie de moyen de connexion s'encliquetant dans une autre partie de moyen de connexion située sur la partie proximale de l'aiguille. Un des inconvénients de cet ensemble est que la connexion ainsi réalisée ne peut être déverrouillée qu'en réalisant l'opération particulière consistant à désengager les deux parties de moyen de connexion l'une de l'autre, nécessitant par exemple l'aide des deux mains. Par ailleurs, cet ensemble peut être utilisé selon trois modes différents de mise en place de la bandelette, à savoir un mode dit par voie basse, un mode dit par voie mixte et un mode dit par voie haute.

Or il serait intéressant de disposer d'un ensemble chirurgical pouvant être utilisé pour d'autres voies d'abord et pour lequel la connexion entre la bande composite et l'aiguille serait libérable facilement.

L'ensemble chirurgical selon l'invention comprend :
a) une bande composite qui rassemble au moins une bandelette de soutènement sous-urétral et une gaine aplatie de protection, ladite bandelette étant disposée, par exemple librement, à l'intérieur de cette gaine ; la bande composite comprend à chacune de ses deux extrémités une même partie, par exemple une même partie femelle, d'un moyen de connexion, verrouillable et déverrouillable ; la gaine aplatie comprend, selon sa longueur, deux parties disposées de part et d'autre d'une zone médiane de séparation ;
b) une aiguille permettant l'engagement et le tirage de la bandelette de soutènement sous-urétral au travers des tissus corporels de la patiente, cette aiguille comprenant au moins une autre partie, par exemple une partie mâle, d'un moyen de connexion, les parties de moyens de connexion de la bande composite et chaque partie de moyen de connexion de l'aiguille permettant de raccorder de manière libérable l'aiguille à au moins l'une des extrémités de ladite bande composite.

La présente invention a pour objet un ensemble chirurgical tel que précédemment défini, à caractère universel, en ce sens qu'il peut être utilisé, quelle que soit la voie d'abord, et la technique d'intervention retenue par le praticien.

Selon l'invention, l'ensemble chirurgical est caractérisé en ce que :
- chaque partie de moyen de connexion que comprend la bande composite inclut une portion de tube déformable élastiquement, et
- chaque partie de moyen de connexion que comprend l'aiguille est conformée pour pouvoir être engagée avec frottement dans ladite portion de tube ou pour pouvoir être insérée avec rétention dans la paroi de cette portion de tube.

Grâce à la portion de tube présente dans les parties de moyen de connexion de la bande composite et aux parties de moyen de connexion spécifiques de l'aiguille, il est possible de réaliser une connexion déverrouillable par simples tractions manuelles antagonistes. La libération de la connexion peut par exemple se faire avec une seule main.

L'aiguille peut comprendre des parties de moyens de connexion à ses extrémités proximale et distale.

Dans une forme préférée de réalisation de l'invention, l'aiguille comprend des moyens de connexion à son extrémité distale.

Ainsi, selon l'ensemble selon l'invention, il est possible de mettre en place la bandelette sous-urétrale selon la voie d'abord dite basse, ou selon la voie d'abord dite mixte, ou selon la voie d'abord dite haute ou encore selon la voie d'abord dite transobturatrice.

En particulier, grâce à l'ensemble selon l'invention, il est possible de mettre en place la bandelette sous-urétrale selon la voie d'abord dite haute ou selon la voie d'abord dite transobturatrice, voies encore appelées « out-in », sans avoir à sectionner ladite bandelette.

De préférence, chaque portion de tube est reliée à l'extrémité correspondante de la bande composite au moyen d'un doigt axial de diamètre légèrement supérieur au diamètre de cette portion de tube, pouvant être engagé dans celle-ci avec frottements.

Chaque doigt peut être pourvu de nervures circulaires en saillie, formant des bossages. Ces bossages favorisent la liaison de ce doigt à ladite portion de tube. Alternativement, le doigt peut être muni d'un pas de vis.

Au moins l'extrémité proximale de l'aiguille peut comprendre une partie terminale conique, de diamètre allant en s'élargissant en direction de la zone médiane de l'aiguille, et une portion cylindrique de diamètre plus faible que le diamètre de la base de la portion conique, cette portion cylindrique délimitant ainsi un épaulement avec la base de la portion conique, la base de ladite portion conique ayant un diamètre supérieur au diamètre interne de la portion de tube et pouvant être engagée avec frottements dans ladite portion de tube, en provoquant un étirement circonférentiel de la paroi de cette portion de tube, ladite portion conique présentant en outre une pente relativement importante et une section réduite, de telle sorte qu'elle est à même de transpercer les tissus corporels de la patiente.

L"aiguille peut être formée par une partie effilée et courbe d'un instrument, apte à être engagé au travers des tissus corporels de la patiente, en passant par le trou obturateur du bassin jusqu'à déboucher au travers du vagin.

Dans ce cas, ladite partie effilée et courbe peut comprendre une portion extérieure rigide et une portion intérieure souple pouvant coulisser le long de la face radialement interne de ladite portion extérieure rigide, ces portion extérieure rigide et portion intérieure souple ayant des extrémités distales ; ladite portion intérieure souple est mobile par rapport à ladite portion extérieure rigide entre une position avancée et une position reculée ; dans ladite position avancée, l'extrémité distale de ladite portion intérieure souple forme, avec l'extrémité distale de ladite portion extérieure rigide, une portion conique à forte pente et à relativement faible section, permettant l'engagement de ladite partie effilée et courbe au travers des tissus corporels de la patiente ; dans la position reculée, l'extrémité distale de la portion intérieure souple se trouve en retrait de l'extrémité distale de la portion extérieure rigide ; l'extrémité distale de la portion extérieure rigide présente un évidement proximal délimitant un rebord à arète vive, lui donnant une conformation en "harpon" et est propre, dans cette position reculée, à pénétrer légèrement dans la paroi de ladite portion de tube lorsque cette portion de tube est engagée sur elle.

La bande composite peut comprendre un moyen de liaison sécable, formé par un fourreau extérieur adhésif, par exemple déchirable par cisaillement, rassemblant les deux extrémités intérieures des deux parties de la gaine aplatie, respectivement adjacentes à la zone de séparation.

La présente invention est maintenant décrite par référence au dessin annexé, dans lequel :
- La figure 1 est une vue de face de la bande composite d'un ensemble chirurgical conforme à invention,
- La figure 2 est une vue côté d'une aiguille pouvant être utilisée avec cette bande composite,
- Les figures 3 et 4 sont des vues respectivement de côté de cette bande composite et de face de la partie proximale de cette aiguille,
- Les figures 5 à 8 sont des vues de quatre étapes successives de mise en place ou mise en oeuvre de cet ensemble chirurgical par une voie d'abord dite "haute",
- Les figures 9A et 9B sont des vues de côté d'un instrument utilisable avec la bande composite pour la mise en place ou mise en oeuvre de l'ensemble chirurgical comprenant cette bande composite et cet instrument par une voie d'abord "transobturatrice", et
- Les figures 10 à 13 sont des vues de quatre étapes successives de mise en place ou mise en oeuvre d'un ensemble chirurgical comprenant cette bande composite et cet instrument, par ladite voie d'abord "transobturatrice".

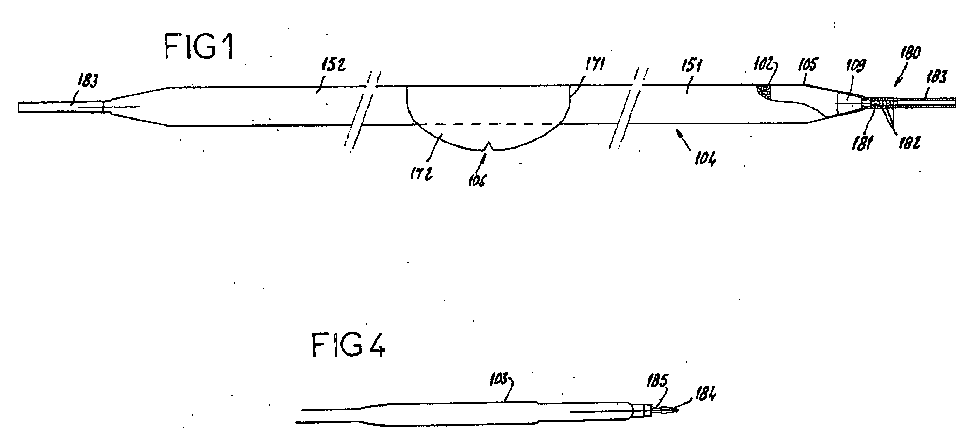

Conformément aux figures 1 à 3, un ensemble chirurgical selon l'invention comprend au minimum :
- une bande composite 104 comportant deux extrémités effilées, de forme triangulaire, à plat,
- et une aiguille 103 comportant une extrémité distale 132 de pénétration et un talon proximal aplati 131.

La bande composite 104 rassemble au moins, une bandelette 102 de soutènement sous-urétral, deux embouts 109, et une gaine aplatie 105 de protection, à l'intérieur de laquelle la bandelette 102 est disposée librement, c'est-à-dire sans être attachée à la gaine 105.

La bandelette 102 de soutènement comprend un tricot ajouré, de préférence indémaillable, obtenu avec des monofilaments ou multifilaments en matériau synthétique biocompatible, par exemple en polypropylène ou polyester.

La bandelette 102 est de préférence formée à partir d'un matériau tricoté macroporeux.

Ce dernier est par exemple un tricot ajouré en monofil de polypropylène ayant une grosseur comprise entre 0,12 et 0,16 millimètre et composé de deux nappes formées par deux barres à passettes enfilées chacune, avec une alternance d'une passette pleine et d'une passette vide, ces deux barres étant déplacées symétriquement en mailles ouvertes suivant le barème suivant :
- barre I : 01-12-32
- barre II : 32-21-01

La bandelette 102 est découpée en longueur dans le sens de la chaîne du tricot. Dans le cas d'une largeur de 12 mm, elle présente les caractéristiques suivantes :
- une résistance à la rupture dans le sens de la chaîne de 105N ± 20%,
- un allongement à la rupture dans le sens de la chaîne de 92 % ±20%,
- un allongement sous 20N de 36 %,
- un début de tuilage avec une force de 6N et un allongement de 15%.

Par "tuilage", on entend l'enroulement spontané de la bandelette 102 sur elle-même, autour de son axe longitudinal, sous contrainte en tension longitudinale.

La bandelette 102 présente des avantages intéressants et en particulier une faible émission de particules lors de son étirement, ainsi qu'un tuilage qui n'apparaît que sous une force importante (6N). Toutes ces caractéristiques précitées n'altèrent en rien la porosité de la bandelette 102.

Cette dernière peut également être réalisée en tout ou en partie en tissu ou matériau biologique résorbable ou rendu non résorbable, par exemple en collagène.

La gaine de protection 105, aplatie, est obtenue à partir d'un matériau synthétique à faible coefficient de frottement, par exemple en PTFE. Cette gaine 105 comprend, selon sa longueur, deux parties 151 et 152 disposées de part et d'autre d'une zone de séparation 106, dans laquelle est disposé un moyen de liaison sécable entre les deux dites parties. Conformément à la figure 1, et selon un mode particulier de réalisation, le moyen de liaison sécable consiste en un fourreau extérieur 171,- adhésif, déchirable, par exemple par cisaillement, au moyen d'un onglet 172. Ce fourreau 171 rassemble deux extrémités intérieures des deux parties 151 et 152 de la gaine 105, respectivement adjacentes à la zone de séparation 106.

La bande composite 104 comprend des moyens de connexion 180 de ses extrémités à l'aiguille 103. Chaque moyen de connexion 180 comprend un doigt axial 181 pourvu de nervures circulaires en saillie 182, formant des bossages, et une portion de tube 183 déformable élastiquement, de diamètre interne légèrement inférieur au diamètre du doigt 181.

Le doigt 181 est destiné à être engagé avec frottements dans la portion de tube 183, les nervures 182 provoquant un étirement circonférentiel de la paroi de cette portion de tube 183, suffisant pour créer une liaison résistante entre le doigt 181 et la portion de tube 183.

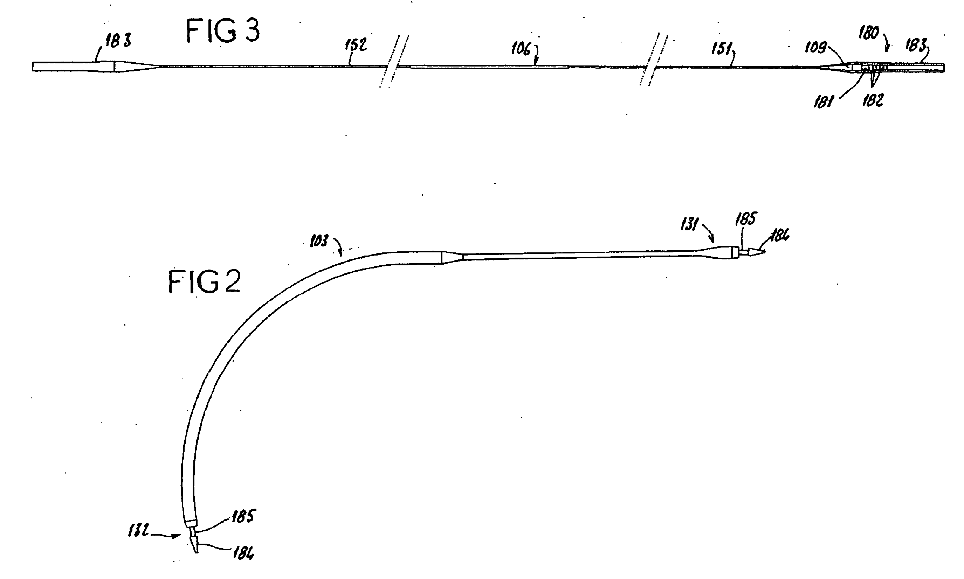

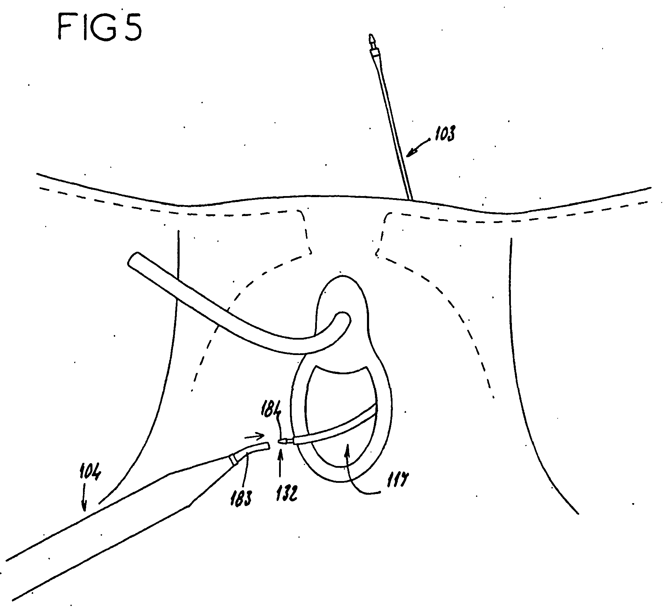

Comme le montrent les figures 2 et 4, l'aiguille 103 comprend des extrémités proximale 131 et distale 132 conformées pour pouvoir être connectées aux portions de tube 183. Pour ce faire, chaque extrémité 131, 132 comprend une partie terminale conique 184, de diamètre allant en s'élargissant en direction de la zone médiane de l'aiguille 103, et une portion cylindrique 185 de diamètre plus faible que le diamètre de la base de la portion conique 184, cette portion cylindrique 185 délimitant ainsi un épaulement avec la portion conique 184.

La base de la portion conique 184 a un diamètre supérieur au diamètre interne de la portion de tube 183 et peut être engagée avec frottements dans la portion de tube 183, en provoquant un étirement circonférentiel de la paroi de cette portion de tube. Cette portion 184 présente en outre une pente relativement importante et a une section réduite, de telle sorte qu'elle est à même de transpercer les tissus corporels de la patiente.

Les portions 184 et 185 et la portion de tube 183 permettent ainsi de réaliser une connexion libérable de la portion de tube 183 à l'une ou l'autre des extrémités 131, 132 de l'aiguille 103 ; cette connexion est suffisamment résistante pour résister à la contrainte de traction qu'exerce la bande 104 sous l'effet des frottements que subit cette bande 104 lors de son engagement dans le corps de la patiente, mais n'est pas suffisamment résistante pour résister à des tractions manuelles antagonistes exercées sur l'aiguille 103 d'une part et sur la portion de tube 183 d'autre part afin de séparer cette aiguille 103 et cette portion de tube 183.

Les portions 184 et 185 de l'extrémité proximale 131 de l'aiguille 103 sont utilisées pour la mise en place de la bande composite 104 par coulissement au travers de trajets corporels appropriés ; les portions 184 et 185 de l'extrémité distale 132 de l'aiguille 103 sont utilisées pour la mise en place de la bande composite 104 selon une voie d'abord dite "haute", montrée sur les figures 5 à 8.

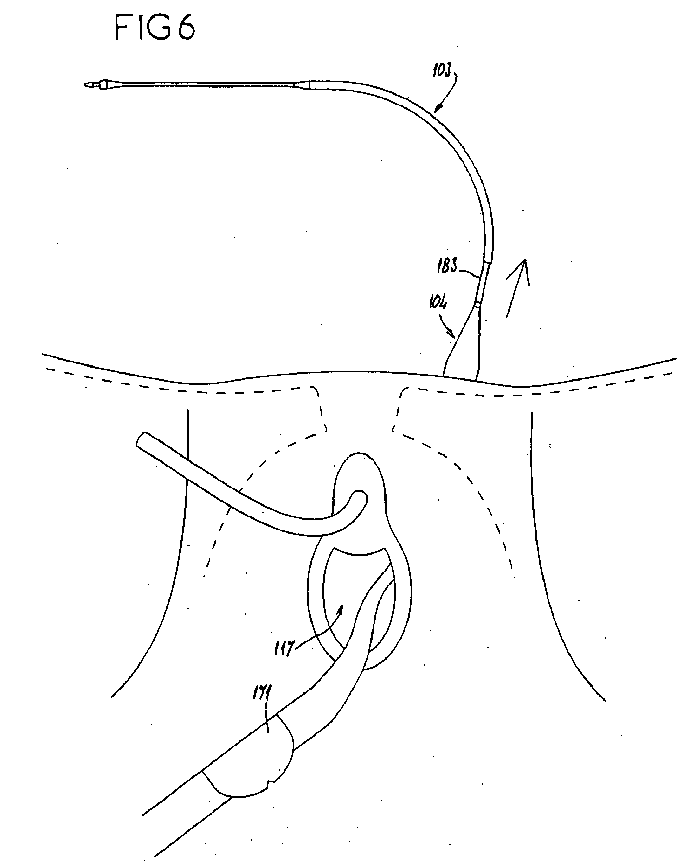

Dans le cas de cette voie d'abord "haute", l'aiguille 103 est engagée au travers de la paroi abdominale, à partir d'une première incision sus-pubienne jusqu'à une incision vaginale, puis l'extrémité distale 132 débouchant du vagin 117 est connectée à la portion de tube 183 de l'une des extrémités de la bande 104 (cf. figure 5). L'aiguille 103 est alors reculée de manière à engager la bande composite 104 au travers du trajet que l'aiguille 103 a permis d'aménager dans le corps de la patiente, jusqu'à faire déboucher la partie d'extrémité de la moitié de la bande 104 reliée à l'aiguille 103 au niveau sus-pubien (cf. figure 6).

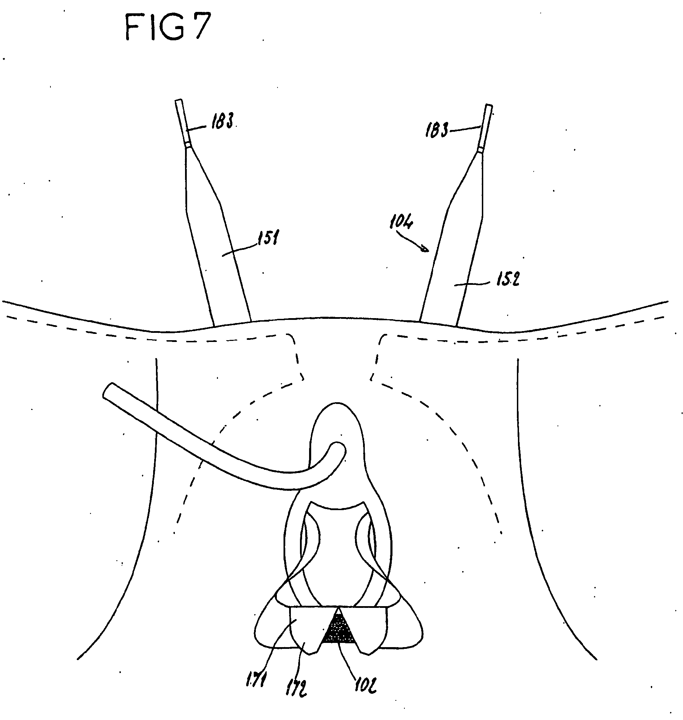

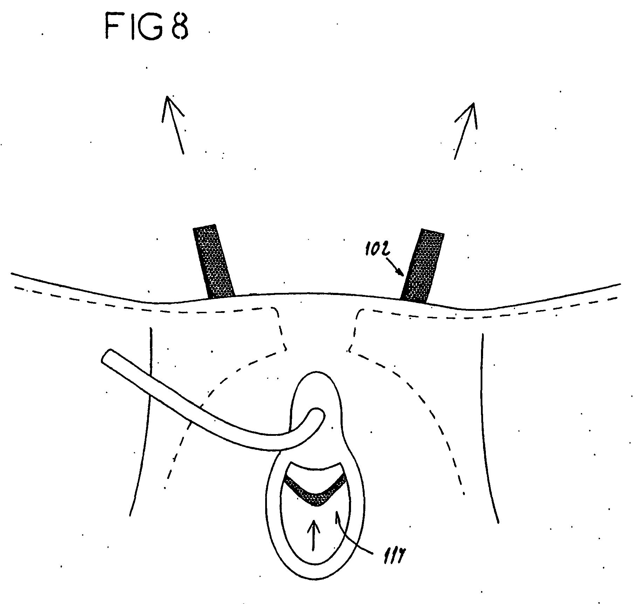

Les mêmes opérations sont effectuées de l'autre côté puis le fourreau 171 est sectionné au moyen de l'onglet 172 (cf. figure 7), après quoi les parties de gaine 151, 152 sont retirées et la bandelette 102 est mise en place (cf. figure 8).

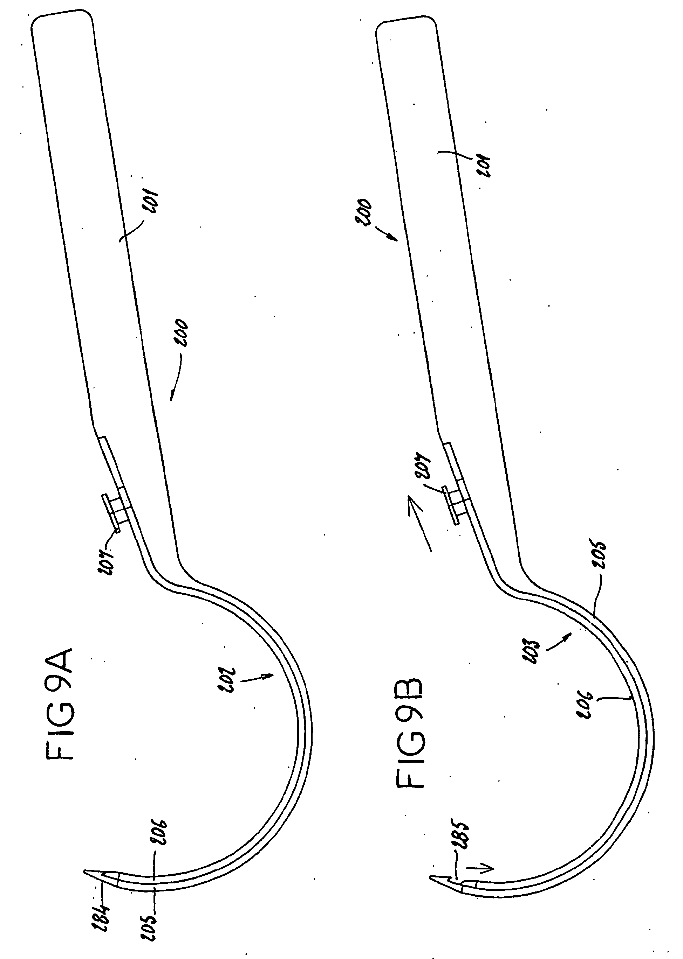

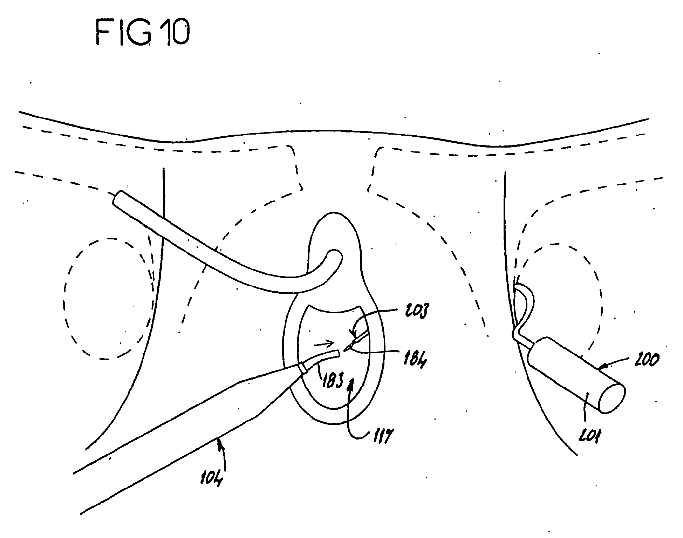

Les figures 9A et 9B montrent un instrument 200 dont une partie 201 forme un manche et une autre partie 203, effilée et courbe, forme une "aiguille" apte à être engagée au travers des tissus corporels de la patiente, en passant par le trou obturateur du bassin jusqu'à déboucher au travers de l'incision vaginale, comme cela apparaît sur la figure 10.

La partie courbe effilée 203 comprend une portion extérieure rigide 205, solidaire du manche 201, et une portion intérieure souple 206. Cette portion intérieure souple 206 peut coulisser le long de la face radialement interne de la portion extérieure 205 et peut être actionnée au moyen d'un bouton de manoeuvre 207 que comprend son extrémité proximale. Des moyens de guidage sont prévus pour guider en coulissement la portion 206 le long de la portion 205, notament sous forme d'une rainure aménagée dans la portion 205 dans laquelle coulisse un patin solidaire de la portion 206.

La portion 206 est mobile par rapport à la portion 205 entre une position avancée montrée sur la figure 9A et une position reculée montrée sur la figure 9B ; dans la position avancée, l'extrémité distale de la portion 206 forme, avec l'extrémité distale de la portion 205, une portion conique 284 à forte pente et à relativement faible section, permettant l'engagement de la partie 203 au travers des tissus corporels de la patiente ; dans la position reculée, l'extrémité distale de la portion 206 se trouve en retrait de l'extrémité distale de la portion 205.

Comme le montre la figure 9B, l'extrémité distale de la portion 205 forme la majeure partie de ladite portion conique 284 mais présente un évidement proximal 285 délimitant un rebord à arète vive. Cette extrémité distale est ainsi conformée en "harpon" et est propre à pénétrer légèrement dans la paroi de la portion de tube 183 lorsque cette portion de tube 183 est engagée sur elle.

L'extrémité distale de la portion 206 présente une forme telle qu'elle occupe ledit évidement proximal en position avancée et qu'elle complète la portion conique 284.

Comme le montrent les figures 10 à 13, la partie 203 est engagée au travers des tissus corporels de la patiente en passant par le trou obturateur du bassin jusqu'à ressortir du vagin 117, la portion 206 étant alors en position avancée. Le bouton 207 est ensuite manoeuvré de manière à reculer la portion 206 puis la portion de tube 183 d'une extrémité de la bande 104 est engagée sur l'extrémité distale en harpon de la portion 205 ; cet engagement permet la pénétration de cette extrémité distale dans la paroi de la portion de tube 183, et permet par conséquent de réaliser une liaison entre cette portion de tube 183 et l'extrémité distale de la portion 205 (cf. figure 10).

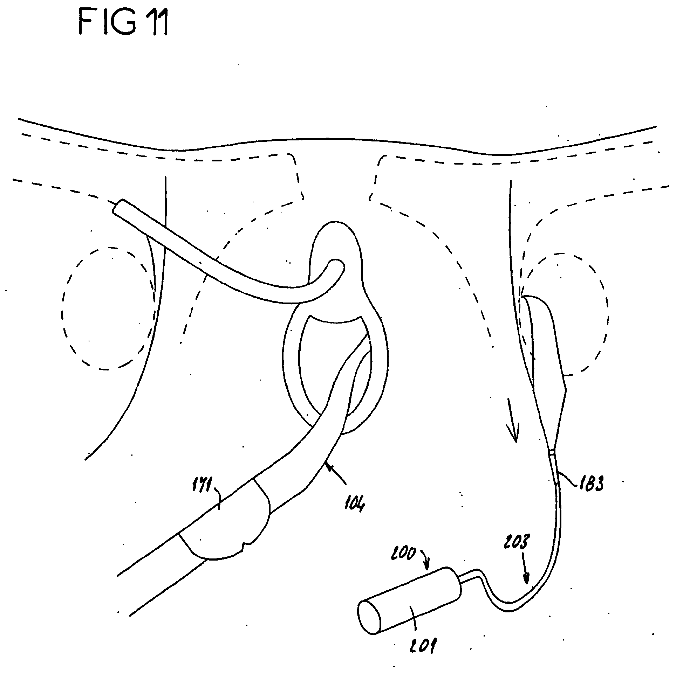

L'instrument 200 est alors retiré de manière à engager la moitié de bande 104 reliée à la partie 203 au travers du trajet corporel aménagé au moyen de cet instrument 200 (cf. figure 11).

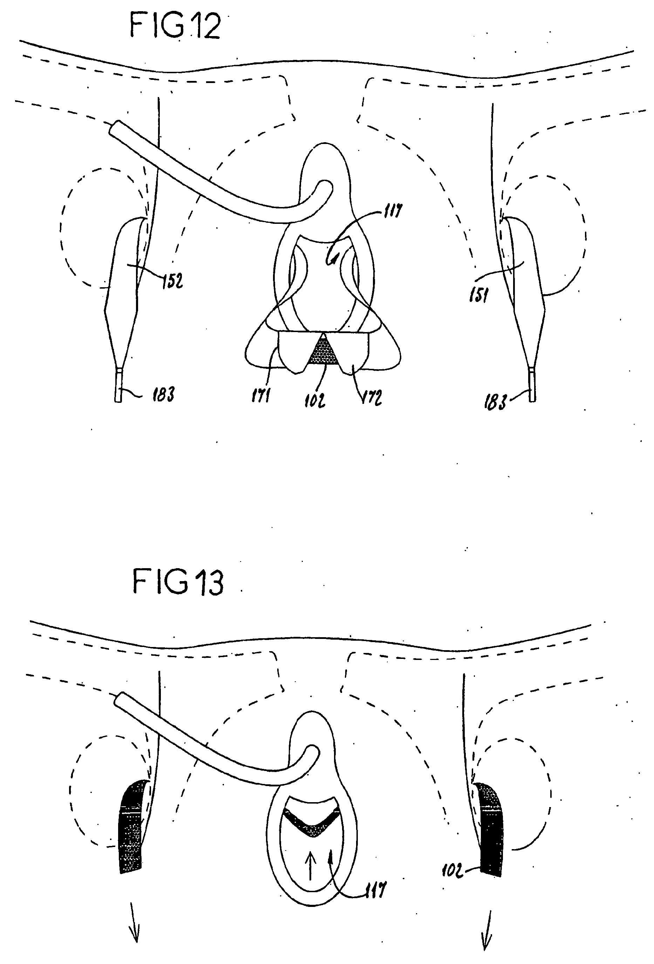

L'extrémité 184 de l'aiguille est déconnectée du tube 183 et les mêmes opérations sont effectuées de l'autre côté. Les deux parties de gaine 151, 152 sont alors séparées et retirées (cf. figure 12) et la bandelette 102 est mise en place par traction sur ses extrémités débouchantes (cf. figure 13).

## Revendications

1. Ensemble chirurgical pour soutenir l'urètre chez la femme, comprenant :
a) une bande composite (104) qui rassemble au moins une bandelette (102) de soutènement sous-urétral et une gaine aplatie (105) de protection, ladite bandelette (102) étant disposée, par exemple librement, à l'intérieur de cette gaine (105) ; la bande composite (104) comprend à chacune de ses deux extrémités une même partie (183), par exemple une même partie femelle, d'un moyen de connexion (183 à 185, 284), verrouillable et déverrouillable ; la gaine (105) aplatie comprend, selon sa longueur, deux parties (151, 152) disposées de part et d'autre d'une zone médiane de séparation ;
b) une aiguille (103, 203), permettant l'engagement et le tirage de la bandelette (102) de soutènement sous-urétral au travers des tissus corporels de la patiente, cette aiguille (103, 203) comprenant au moins une autre partie (184, 185, 284), par exemple une partie mâle, d'un moyen de connexion, les moyens de connexion de la bande composite (104) et chaque moyen de connexion de l'aiguille (103, 203) permettant de raccorder de manière libérable l'aiguille (103, 203) à au moins l'une des extrémités de ladite bande composite (104) ;
ensemble **caractérisé en ce que** :
- chaque partie (183) de moyen de connexion que comprend la bande composite (104) inclut une portion de tube (183) déformable élastiquement, et
- chaque partie (184, 185, 284) de moyen de connexion que comprend l'aiguille (103, 203) est conformée pour pouvoir être engagée avec frottement dans ladite portion de tube (183) ou pour pouvoir être insérée avec rétention dans la paroi de cette portion de tube (183).

2. Ensemble selon la revendication 1, **caractérisé en ce que** l'aiguille (103) comprend des parties (184, 185) de moyens de connexion à ses extrémités proximale et distale.

3. Ensemble selon la revendication 1, **caractérisé en ce que** chaque portion de tube (183) est reliée à l'extrémité correspondante de la bande composite (104) au moyen d'un doigt axial (181) de diamètre légèrement supérieur au diamètre de cette portion de tube (183), pouvant être engagé dans celle-ci avec frottements.

4. Ensemble selon la revendication 3, **caractérisé en ce que** chaque doigt (181) est pourvu de nervures circulaires (182) en saillie, formant des bossages.

5. Ensemble selon la revendication 1, **caractérisé en ce qu'**au moins l'extrémité proximale de l'aiguille (103) comprend une partie terminale conique (184), de diamètre allant en s'élargissant en direction de la zone médiane de l'aiguille (103), et une portion cylindrique (185) de diamètre plus faible que le diamètre de la base de la portion conique (184), cette portion cylindrique (185) délimitant ainsi un épaulement avec la base de la portion conique (184), la base de ladite portion conique (184) ayant un diamètre supérieur au diamètre interne de la portion de tube (183) et pouvant être engagée avec frottements dans cette portion de tube (183), en provoquant un étirement circonférentiel de la paroi de cette portion de tube (183), ladite portion conique (184) présentant en outre une pente relativement importante et une section réduite, de telle sorte qu'elle est à même de transpercer les tissus corporels de la patiente.

6. Ensemble selon la revendication 1, **caractérisé en ce que** l'aiguille (203) est formée par une partie effilée et courbe d'un instrument (200), apte à être engagé au travers des tissus corporels de la patiente, en passant par le trou obturateur du bassin jusqu'à déboucher au travers du vagin.

7. Ensemble selon la revendication 6, **caractérisé en ce que** ladite partie effilée et courbe (203) comprend une portion extérieure rigide (205) et une portion intérieure souple (206) pouvant coulisser le long de la face radialement interne de ladite portion extérieure rigide (205), ces portion extérieure rigide (205) et portion intérieure souple (206) ayant des extrémités distales ; ladite portion intérieure souple (206) est mobile par rapport à ladite portion extérieure rigide (205) entre une position avancée et une position reculée ; dans ladite position avancée, l'extrémité distale de ladite portion intérieure souple (206) forme, avec l'extrémité distale de ladite portion extérieure rigide (205), une portion conique (284) à forte pente et à relativement faible section, permettant l'engagement de ladite partie effilée et courbe (203) au travers des tissus corporels de la patiente ; dans la position reculée, l'extrémité distale de la portion intérieure souple (206) se trouve en retrait de l'extrémité distale de la portion extérieure rigide (205) ; l'extrémité distale de la portion extérieure rigide (205) présente un évidement proximal délimitant un rebord à arète vive, lui donnant une conformation en "harpon" et est propre, dans cette position reculée, à pénétrer légèrement dans la paroi de ladite portion de tube (183) lorsque cette portion de tube (183) est engagée sur elle.

8. Ensemble selon la revendication 1, **caractérisé en ce que** la bande composite (104) comprend un moyen de liaison sécable, formé par un fourreau extérieur adhésif (171), par exemple déchirable par cisaillement, rassemblant les deux extrémités intérieures des deux parties (151, 152) de la gaine (105) aplatie, respectivement adjacentes à la zone de séparation.

9. Ensemble selon la revendication 1, **caractérisé en ce que** la bandelette (102) de soutènement comprend un tissu tricoté ajouré indémaillable, obtenu avec des monofilaments ou multifilaments en matériau synthétique biocompatible, par exemple en polypropylène ou polyester.

10. Ensemble selon la revendication 1, **caractérisé en ce que** la bandelette (102) de soutènement est réalisée en tout ou en partie en tissu ou matériau biologique, par exemple en collagène.

11. Ensemble selon la revendication 1, **caractérisé en ce que** la gaine (105) de protection est obtenue à partir d'un matériau synthétique à faible coefficient de frottement, par exemple en PTFE.

12. Ensemble selon la revendication 1, **caractérisé en ce que** l'aiguille (103, 203) comprend des parties (184, 185, 284) de moyens de connexion à son extrémité distale.

## Claims

1. Surgical assembly for supporting the female urethra, comprising:
a) a composite band (104) which includes at least one suburethral support strip (102) and a flat protective stmaLh (105), said strip (102) being arranged, for example freely, inside this sheath (105); both ends of the composite band (104) comprise a common part (183), for example a female common part, of a connecting means (183 to 185, 284) which can be locked and unlocked; the flat sheath (105) comprises, along its length, two parts (151, 152) arranged on either side of a middle zone of separation;
b) a needle (103, 203), allowing the suburethral support strip (102) to be engaged and pulled through the body tissues of the patient, this needle (103, 203) comprising at least one other part (184, 185, 284), for example a male part, of a connecting means, the connecting means of the composite band (104) and each connecting means of the needle (103, 203) making it possible to connect the needle (103, 203) releasably to at least one of the ends of said composite band (104);
said assembly being **characterized in that**:
- each part (183) of the connecting means belonging to the composite band (104) includes an elastically deformable tube portion (183), and
- each part (184, 185, 284) ot the connecting means belonging to the needle (103, 203) is shaped so that it can be engaged with friction in said tube portion (183) or can be inserted with retention in the wall of this tube portion (183).

2. Assembly according to Claim 1, **characterized in that** the needle (103) comprises parts (184, 185) of connecting means at its proximal and distal ends.

3. Assembly according to Claim 1, **characterized in that** each tube portion (183) is connected to the corresponding end of the composite band (104) by means of an axial finger (181) with a diameter slightly greater than the diameter of this tube portion (183), and able to be engaged in the latter with friction.

4. Assembly according to Claim 3, **characterized in that** each finger (181) is provided with protruding circular ribs (182) forming bosses.

5. Assembly according to Claim 1, **characterized in that** at least the proximal end of the needle (103) comprises a conical end part (184), with a diameter widening in the direction of the middle zone of the needle (103), and a cylindrical portion (185) of smaller diameter than the diameter of the base of the conical portion (184), this cylindrical portion (185) thus delimiting a shoulder with the base of the conical portion (184), the base of said conical portion (184) having a diameter greater than the internal diameter of the tube portion (183) and being able to be engaged with friction in this tube portion (183), causing a circumferential stretching of the wall of this tube portion (183), said conical portion (184) additionally having a relatively pronounced slope and a reduced cross section, in such a way that it is able to pierce through the body tissues of the patient.

6. Assembly according to Claim 1, **characterized in that** the needle (203) is formed by a tapered and curved part of an instrument (200) able to be engaged through the body tissues of the patient, by passing through the obturator foramen of the pelvis and emerging through the vagina.

7. Assembly according to Claim 6, **characterized in that** said tapered and curved part (203) comprises a rigid outer portion (205) and a flexible inner portion (206) which is able to slide along the radially inner face of said rigid outer portion (205), said rigid outer portion (205) and flexible inner portion (206) having distal ends; said flexible inner portion (206) is movable with respect to said rigid outer portion (205) between an advanced position and a retracted position; in said advanced position, the distal end of said flexible inner portion (206) forms, with the distal end of said rigid outer portion (205), a conical portion (284) with pronounced slope and relatively small cross section, permitting engagement of said tapered and curved part (203) through Lhe body tissues of the patient; in the retracted position, the distal end of the flexible inner portion (206) is situated set back from the distal end of the rigid outer portion (205); the distal end of the rigid outer portion (205) has a proximal recess delimiting a sharp-edged shoulder, giving it a "harpoon" configuration, and is able, in this retracted position, to penetrate slightly into the wall of said tube portion (183) when this tube portion (183) is engaged on it.

8. Assembly according to Claim 1, **characterized in that** the composite band (104) comprises a breakable linking means formed by an adhesive outer sleeve (171), which for example can be torn off by shearing, which joins the two inner ends of the two parts (151, 152) of the flat sheath (105) respectively adjacent to the zone of separation.

9. Assembly according to Claim 1, **characterized in that** the support strip (102) comprises an openwork knit which is unable to unravel and is obtained from monofilaments or multifilaments of a biocompatible synthetic material, for example polypropylene or polyester.

10. Assembly according to Claim 1, **characterized in that** the support strip (102) is made wholly or partly of biological tissue or material, for example collagen.

11. Assembly according to Claim 1, **characterized in that** the protective sheath (105) is obtained from a synthetic material with a low coefficient of friction, for example PTFE.

12. Assembly according to Claim 1, **characterized in that** the needle (103, 203) comprises parts (184, 185, 284) of connecting means at its distal end.

## Patentansprüche

1. Chirurgische Anordnung zum Stützen der Urethra bei der Frau, mit:
a) einem Kompositstreifen (104), der zumindest einen schmalen Streifen (102) zum suburethralen Stützen und eine abgeflachte Schutzhülle (105) aufweist, wobei der schmale Streifen (102), beispielsweise frei, im Inneren der Hülle (105) angeordnet ist, wobei der Kompositstreifen (104) an seinen beiden Enden einen gleichen Bereich (183), beispielsweise einen weiblichen Bereich, eines Verbindungsmittels (183 bis 185, 284) aufweist, das verriegelbar und cntriegelbar ist, wobei die abgeflachte Hülle (105) in ihrer Längsrichtung zwei Bereiche (151, 152) aufweist, die beidseits einer mittleren Trennungszone angeordnet sind;
b) einer Nadel (103, 203), die einen Eingriff mit dem schmalen Streifen (102) zur suburethralen Stützung und ein Ziehen des schmalen Streitcns (102) zur suburethralen Stützung durch Körpergewebe der Patientin hindurch ermöglicht, wobei die Nadel (103, 203) zumindest einen weiteren Bereich (184, 185, 284), beispielsweise einen männlichen Bereich, eines Verbindungsmittels aufweist, wobei die Verbindungsmittel des Kompositstreifens (104) und jedes Verbindungsmittel der Nadel (103, 203) eine lösbare Verbindung der Nadel (103, 203) mit zumindest einem der Enden des Kompositstreifens (104) ermöglichen;
wobei die Anordnung **dadurch gekennzeichnet ist, dass**:
- jeder Bereich (183) des Verbindungsmittels, das der Kompositstreifen (104) autweist, einen elastisch verformbaren Rohrabschnitt (183) aufweist, und
- jeder Bereich (184, 185, 284) des Verbindungsmittels, das die Nadel (103, 203) aufweist, so ausgestaltet ist, dass er in Reibeingriff mit dem Rohrabschnitt (183) gebracht oder unter Rückhaltung in der Wand des Rohrabschnittes (103) eingesetzt werden kann.

2. Anordnung nach Anspruch 1, **dadurch gekennzeichnet, dass** die Nadel (103) die Bereiche (184, 185) der Verbindungsmittel an ihrem proximalen und distalen Ende aufweist.

3. Anordnung nach Anspruch 1, **dadurch gekennzeichnet, dass** jeder Rohrabschnitt (183) mit dem entsprechenden Ende des Kompositstreifens (104) mittels eines axialen Fingers (181) mit geringfügig größerem Durchmesser als der Durchmesser des Rohrabschnitts (183) verbunden ist, das mit diesem in Reibeingriff gebracht werden kann.

4. Anordnung nach Anspruch 3, **dadurch gekennzeichnet, dass** jeder Finger (181) mit kreisförmigen vorspringenden Rippen (182) versehen ist, die Erhebungen bilden.

5. Anordnung nach Anspruch 1, **dadurch gekennzeichnet, dass** zumindest das proximale Ende der Nadel (103) einen konischen Endbereich (184), dessen Durchmesser sich in Richtung zur mittleren Zone der Nadel (103) vergrößert, und einen zylindrischen Abschnitt (185) mit geringerem Durchmesser als der Durchmesser der Basis des konischen Abschnitts (184) aufweist, so dass der zylindrische Abschnitt (185) eine Stufe mit der Basis des konischen Abschnitts (184) definiert, wobei die Basis des konischen Abschnitts (184) einen Durchmesser aufweist, der größer ist als der Innendurchmesser des Rohrabschnitts (183) und mit diesem Rohrabschnitt (183) in Reibeingriff kommen kann, um eine umfängliche Dehnung der Wand des Rohrabschnitts (183) hervorzurufen, wobei der konische Abschnitt (184) außerdem eine relativ starke Neigung und einen verminderten Querschnitt aufweist, derart, dass er in der Lage ist, Körpergewebe der Patienten zu durchstechen.

6. Anordnung nach Anspruch 1, **dadurch gekennzeichnet, dass** die Nadel (203) durch einen verjüngten und gekrümmten Bereich eines Instruments (200) gebildet ist, das in der Lage ist, durch Körpergewebe der Patientin hindurchzugreifen, wobei es durch das Foramen obturatum des Beckens hindurchgeht, bis es aus der Vagina heraustritt.

7. Anordnung nach Anspruch 6, **dadurch gekennzeichnet, dass** der verjüngte und gekrümmte Bereich (203) einen starren äußeren Abschnitt (205) und einen biegsamen inneren Abschnitt (206) aufweist, der entlang der radial inneren Seite des starren äußeren Abschnitts (205) gleiten kann, wobei der starre äußere Abschnitt (205) und der biegsame innere Abschnitt (206) distale Enden aufweisen, wobei der biegsame innere Abschnitt (206) bezüglich dem starren äußeren Abschnitt (205) zwischen einer vorgeschobenen Stellung und einer zurückgezogenen Stellung beweglich ist, wobei in der vorgeschobenen Stellung das distale Ende des biegsamen inneren Abschnitts (206) mit dem distalen Ende des starren äußeren Abschnitts (205) einen konischen Abschnitt (284) mit starker Neigung und relativ geringem Querschnitt bildet, was den Durchgriff des verjüngten und gekrümmten Bereichs (203) durch Körpergewebe der Patientin hindurch ermöglicht, wobei sich das distale Ende des biegsamen inneren Abschnitts (206) in der zurückgezogenen Stellung von dem distalen Ende des starren äußeren Abschnitts (205) entfernt befindet, wobei das distale Ende des starren äußeren Abschnitts (205) eine proximale Ausnehmung aufweist, die einen scharfkantigen Rand begrenzt, der ihm die Gestalt einer "Harpune" verleiht und geeignet ist, in der zurückgezogenen Stellung leicht in die Wand des Rohrabschnitts (183) einzudringen, wenn der Rohrabschnitt (183) mit ihm in Eingriff steht.

8. Anordnung nach Anspruch 1, **dadurch gekennzeichnet, dass** der Kompositstrcifen (104) ein Mittel zur teilbaren Verbindung aufweist, das durch eine äußere klebende Lasche (171) gebildet ist, die beispielsweise durch Abscherung abreißbar ist, und die die beiden inneren Enden der beiden Bereiche (151, 152) der abgeflachten Hülle (105), jeweils der Trennungszone benachbart, verbindet.

9. Anordnung nach Anspruch 1, **dadurch gekennzeichnet, dass** der schmale Streifen (102) zum Stützen ein gewirktcs durchbrochenes maschenfestes Gewebe aufweist, das mit Monofilamenten oder Multifilamenten aus einem biokompatiblen synthetischen Material, beispielsweise aus Polypropylen oder Polyester, gefertigt ist.

10. Anordnung nach Anspruch 1, **dadurch gekennzeichnet, dass** der schmale Streifen (102) zum Stützen ganz oder teilweise aus einem biologischen Material, beispielsweise aus Collagen, gefertigt ist.

11. Anordnung nach Anspruch 1, **dadurch gekennzeichnet, dass** die Schutzhülle (105) auf der Basis eines synthetischen Materials mit geringem Reibungskoeffizienten, beispielsweise aus PTFE, gefertigt ist.

12. Anordnung nach Anspruch 1, **dadurch gekennzeichnet, dass** die Nadel (103, 203) die Bereiche (184, 185, 284) der Verbindungsmittel an ihrem distalen Ende aufweist.
